# EUROPÄISCHE PATENTSCHRIFT

(11) **EP 0 751 111 B1**
(45) Veröffentlichungstag und Bekanntmachung des Hinweises auf die Patenterteilung: **13.09.2000**
(21) Anmeldenummer: 96109473.7
(22) Anmeldetag: 13.06.1996
(51) Int. Cl.: C07C 45/68, C07C 49/683, C07C 49/697

(54) **Verfahren zur Herstellung von 2,2-Dialkylaryliden-cycloalkanonen**
Process for preparing 2,2-dialkylaryliden-cycloalkanones
Procédé pour la préparation des 2,2-dialkylearylidène-cycloalcanones

(30) Priorität: 28.06.1995 DE 19523449
(43) Veröffentlichungstag der Anmeldung: 02.01.1997
(73) Patentinhaber: BAYER AG, 51368 Leverkusen (DE)
(72) Erfinder: Müller, Nikolaus, Dr., 40789 Monheim (DE); Essert, Thomas, Dr., 51491 Overath (DE)

(56) Entgegenhaltungen:
- EP-A- 0 712 836
- J. AMER. CHEM. SOC., Bd. 87, 1968, Seiten 82-86, XP002035146 S. BOATMAN ET AL.: "Alkylations at the alpha'-methylene or methinyl group of alpha-formyl cyclic ketones through their dicarbanions. Angular alkylations"

## Beschreibung

Die vorliegende Erfindung betrifft ein besonders vorteilhaftes Verfahren zur Herstellung von 2,2-Dialkyl-aryliden-cycloalkanonen aus den entsprechenden 2-Alkyl-aryliden-cycloalkanonen durch Alkylierung.

Es ist bereits bekannt, 2,2-Dialkyl-aryliden-cycloalkanone durch Umsetzung von 1-Alkyl-aryliden-cycloalkanonen mit Alkylhalogeniden in Gegenwart von sehr starken Basen herzustellen. So beschreibt EP-A1 378 953 die Herstellung von 2,2-Diallyl-5-(4-chlorbenzyliden)-cyclopentanon durch Umsetzung von 2-Allyl-5-(4-chlorbenzyliden)-cyclopentanon mit Allylchlorid in Gegenwart von Natriumhydrid in Toluol/tert.-Amylalkohol und die Herstellung von 2,2-Dimethyl-6-(4-chlorbenzyliden)-cyclohexanon aus 2-Methyl-6-(4-chlorbenzyliden)-cyclohexanon durch Alkylierung mit Methyliodid in Gegenwart von Toluol/tert.-Amylalkohol und Natriumhydrid.

Die Einführung eines n-Butylrestes mit n-Butylbromid in 2-Methyl-6-hydroxymethylen-cyclohexanon gelingt ebenfalls nur mit der sehr starken Base Kaliumamid in Ammoniak (siehe Org. Synth. 48, 40 (1968) und J. Am. Chem. Soc. 87, 82 (1968)). Die beschriebenen Verbindungen haben den Nachteil, daß wie bei allen schwächer methylenaktiven Verbindungen sehr starke Basen wie Alkaliamide, Alkalihydride, Triphenylmethide der Alkalimetalle oder tertiäre Alkoxide eingesetzt werden müssen. Der Umgang mit solchen Basen im technischen Maßstab ist wegen der hohen Reaktivität schwierig, und es müssen spezielle Maßnahmen, wie das Arbeiten unter Inertgas oder der Einsatz spezieller Lösungsmittel ergriffen werden. Außerdem sind diese Basen wegen ihrer aufwendigen Herstellung teuer, so daß ihr Einsatz unwirtschaftlich ist.

Die am häufigsten Verwendung findenden Alkalihydride, vor allem Natriumhydrid, müssen wegen der Entwicklung von Wasserstoff mit besonderer Vorsicht gehandhabt werden.

Die in der Literatur erwähnten Alkalimetall-tert.-alkoxide, die in dem entsprechenden Alkohol als Lösungsmittel eingesetzt werden können, haben den gravierenden Nachteil, daß zur Alkylierung die Ketoverbindung vollständig in ihr Enolat überführt werden muß und die Basizität der tertiären Alkoxide hierzu meist nicht ausreicht. Deshalb finden Nebenreaktionen zwischen dem Enolation und freiem Keton statt, z.B. Aldolkondensationen und Michael-Additionen, die zu erheblichen Ausbeuteverlusten führen und aufwendige Reinigungsoperationen notwendig machen (siehe Bull. Soc. Chim. Franc. 533-537 (1950) und 1040 (1956)).

Triphenylmethylide der Alkalimetalle haben vor allem den Nachteil eines hohen Zwangsanfalls an Triphenylmethan. Das in einigen Fällen eingesetzte Natriumsalz von Dimethylsulfoxid hat den Nachteil, daß es als Carbanion selbst an die Carbonylgruppe addiert und auch selbst alkyliert werden kann. Außerdem muß diese Verbindung in einer vorgeschalteten Reaktion aus Dimethylsulfoxid und Natriumhydrid zuerst gebildet werden, wobei man wieder vor dem Problem des Einsatzes von Alkalihydriden steht (siehe J. Am. Chem. Soc. 87, 1345 (1965) und 84, 866 (1962)).

2,2-Dialkyl-aryliden-cycloalkanone können weiterhin durch Aldolkondensation von 2,2-dialkylsubstituierten Cycloalkanonen mit aromatischen Aldehyden hergestellt werden. So ist in der EP-A1 467 791 und der EP-A1 467 792 die Herstellung von 2,2-Dimethyl-5-(4-chlorbenzyliden)-cyclopentanon beschrieben. Die Probleme dieses an sich einfachen Verfahrens liegen in der schwierigen Zugänglichkeit der Ausgangsmaterialien. 2,2-Dialkylcycloalkanone müssen wegen des Vorhandenseins zweier methylenaktiver α-Positionen reversibel blockiert werden, was zwei zusätzliche Reaktionsstufen erfordert (siehe Org. Synth. 48, 40 (1968)). Als Alternative für die Herstellung von 2,2-Dialkylcycloalkanonen ist die Cyclisierung von 2,2-Dialkyl-α,ω-dicarbonsäureestern oder die thermische Cyclisierung der entsprechenden Carbonsäuren unter Katalyse von Metallsalzen beschrieben (siehe EP-A1 626 363)). Hier wird das Problem aber auf die Zugänglichkeit der 2,2-dialkylsubstituierten Dicarbonsäuren oder deren Ester verlagert, die im Vergleich zu den 2,2-dialkylsubstituierten Cycloalkanonen noch schwieriger ist.

Es wurde nun ein Verfahren zur Herstellung von 2,2-Dialkyl-aryliden-cycloalkanonen der Formel 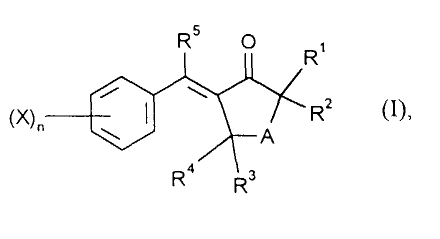 in der
- A: für gegebenenfalls substituiertes -(CH₂)-ₓ mit x = 1, 2 oder 3 steht,
- R¹ und R²: für gleiche oder verschiedene, gegebenenfalls substituierte C₁-C₄-Alkyl- oder C₃-C₇-Cycloalkylreste stehen,
- R³, R⁴ und R⁵: gleich oder verschieden sind und für Wasserstoff oder gegebenenfalls substituiertes C₁-C₄-Alkyl oder C₃-C₇-Cycloalkyl stehen,
- X: für Halogen, Cyano, Nitro. C₁-C₄-Alkyl, C₁-C₄-Halogenalkyl, C₁-C₄-Alkoxy oder C₁-C₄-Halogenalkoxy stehen, wobei beim Vorhandensein mehrerer X-Reste diese gleich oder verschieden sein können, und
- n: für null oder eine ganze Zahl von 1 bis 5 steht,
gefunden, das dadurch gekennzeichnet ist, daß man 2-Alkyl-aryliden-cycloalkanone der Formel 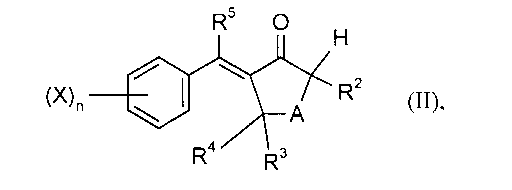 in der die verwendeten Symbole die bei Formel (I) angegebene Bedeutung haben, in Gegenwart von Metallhydroxiden und tertiären Alkoholen mit Alkylhalogeniden der Formel (III)

R¹-H (III),

in der
- R¹: die oben angegebene Bedeutung hat und
- Y: für Chlor, Brom oder Iod steht, umsetzt.
- A: kann gegebenenfalls einen oder mehrere, gleiche oder verschiedene Substituenten aus der Gruppe C₁-C₄-Alkyl und C₃-C₇-Cycloalkyl enthalten, wobei C₁-C₄-Alkyl seinerseits gegebenenfalls durch Halogen oder C₁-C₄-Alkoxy und C₃-C₇-Cycloalkyl seinerseits gegebenenfalls durch Halogen, C₁-C₄-Alkyl oder C₁-C₄-Alkoxy substituiert sein kann. Vorzugsweise steht A für -CH₂-, -CH₂-CH₂- oder -CH₂-CH₂-CH₂-, d.h. vorzugsweise werden 2-Alkyl-aryliden-cyclopentanone, -cyclohexanone oder -cycloheptanone der Formel (II) zu 2,2-Dialkyl-5-aryliden-cyclopentanonen, 2,2-Dialkyl-6-arylidencyclohexanonen oder 2,2-Dialkyl-7-aryliden-cycloheptanonen der Formel (I) umgesetzt.

Soweit R¹ und R² für C₁-C₄-Alkyl stehen, kann dieses gegebenenfalls durch Halogen, C₁-C₄-Alkoxy, Halogen-C₁-C₄-alkoxy, C₂-C₄-Alkenyl, C₂-C₄-Alkinyl, Halogen-C₂-C₄-alkenyl oder Halogen-C₂-C₄-alkinyl substituiert sein. Soweit R¹ und R² für C₃-C₇-Cycloalkyl stehen, kann dieses durch Halogen, C₁-C₄-Alkoxy oder C₁-C₄-Alkyl substituiert sein. Vorzugsweise stehen R¹ und R² unabhängig voneinander für unsubstituiertes C₁-C₄-Alkyl oder unsubstituiertes C₂-C₄-Alkenyl.

Soweit R³, R⁴ und R⁵ für C₁-C₄-Alkyl stehen, kann dieses gegebenenfalls durch Halogen oder C₁-C₄-Alkoxy substituiert sein. Soweit R³, R⁴ und R⁵ für C₃-C₇-Cycloalkyl stehen, kann dieses gegebenenfalls durch Halogen, C₁-C₄-Alkyl oder C₁-C₄-Alkoxy substituiert sein. Vorzugsweise stehen R³, R⁴ und R⁵ für Wasserstoff.
- X: steht vorzugsweise für Fluor, Chlor, Cyano oder C₁-C₄-Alkyl.
- n: steht vorzugsweise für null, 1 oder 2. Im Falle n = 1 befindet sich X vorzugsweise in 4-Stellung. Im Falle n = 2 befindet sich X vorzugsweise in 2- und 4-Stellung.

Halogen kann in allen Bedeutungen, außer bei Alkylhalogeniden, beispielsweise Fluor, Chlor oder Brom bedeuten. Vorzugsweise steht es für Fluor oder Chlor.

Besonders bevorzugte Ausgangsverbindungen der Formel (II) sind: 2-Methyl- und 2-Ethyl-5-(4-fluor-, 4-chlor-, 4-cyano- und 3,4-dichlor-benzyliden)-cyclopentanon, -cyclohexanon und -cycloheptanon.

2-Alkyl-aryliden-cycloalkanone der Formel (II) sind bekannte Verbindungen oder analog bekannten Verbindungen herstellbar.

Ein bevorzugtes Verfahren zur Herstellung von 2-Alkyl-aryliden-cycloalkanonen der Formel (II) wird beispielhaft an der Herstellung von 2-Alkyl-aryliden-cyclopentanonen wie folgt erläutert: Zunächst wird ein 2-Alkylcyclopentanon hergestellt, in dem man Adipinsäureester mit einem Alkoholat umsetzt, so daß man das Salz eines Cyclopentanon-2-carbonsäureesters erhält, dieses ohne vorherige Isolierung alkyliert, so einen 2-Alkyl-cyclopentanon-2-carbonsäureester erhält und diesen ohne vorherige Isolierung durch die Behandlung mit einem Säure und Erhitzen decarboxyliert. Das so erhaltene 2-Alkyl-2-cyclopentanon wird dann mit einer aromatischen Carbonylverbindung in Gegenwart eines basischen Katalysators umgesetzt. Diese Arbeitsweise ist Gegenstand anderer Patentanmeldungen.

Bevorzugt sind Alkylhalogenide der Formel (III), bei denen R¹ die oben angegebene bevorzugte Bedeutung hat und Y für Chlor oder Brom steht. Besonders bevorzugte Alkylhalogenide der Formel (III) sind: Methylchlorid, Methylbromid, Ethylchlorid, Ethylbromid, n-Propylchlorid, iso-Propylchlorid, n-Propylbromid, iso-Propylbromid, 1-Chlorbutan, 2-Chlorbutan, Isobutylchlorid, 1-Brombutan, 2-Brombutan. Isobutylbromid, Allylchlorid, Allylbromid, Crotylchlorid, Crotylbromid, Propargylchlorid und Propargylbromid.

Es ist bevorzugt zusätzlich zu Alkylhalogeniden der Formel (III) mit Y = Chlor, Alkaliiodide einzusetzen, bevorzugt Kalium- und/oder Natriumiodid. Bezogen auf eingesetzte Verbindung der Formel (II) kann man z.B. 0,1 bis 50 Mol-%, vorzugsweise 1 bis 25 Mol-% und besonders bevorzugt 2,5 bis 10 Mol-% Alkaliiodide einsetzen.

Alkylhalogenide werden im allgemeinen in mindestens stöchiometrischer Menge eingesetzt, beispielsweise pro Mol eingesetzte Verbindung der Formel (II) 1 bis 30 Mole Alkylhalogenid. Vorzugsweise beträgt diese Menge 1,2 bis 10 Mol, insbesondere 1,3 bis 2,5 Mol.

Als Metallhydroxide können z.B. Alkali- und Erdalkalihydroxide wie Lithium-, Natrium-, Kalium-, Cäsium-, Magnesium-, Calcium-, Strontium- und Bariumhydroxid eingesetzt werden. Bevorzugt sind Natrium-, Kalium-, Magnesium- und Caiciumhydroxid; besonders bevorzugt ist Kaliumhydroxid.

Metallhydroxide werden im allgemeinen in mindestens äquimolarer Menge, vorzugsweise im Überschuß eingesetzt. Pro Mol eingesetzter Verbindung der Formel (II) kann man z.B. 1,09 bis 10 Äquivalente Metallhydroxid einsetzen. Vorzugsweise beträgt diese Menge 1,1 bis 5 Äquivalente, insbesondere 1,2 bis 2,5 Äquivalente.

Als tertiäre Alkohole können beispielsweise solche der Formel (IV) eingesetzt werden 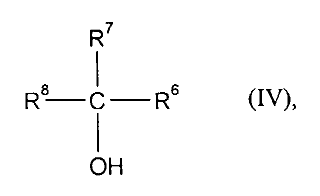 in der R⁶, R⁷ und R⁸ gleich oder verschieden sind und jeweils für C₁-C₄-Alkyl, C₂-C₄-Alkenyl, C₂-C₄-Alkinyl, C₃-C₇-Cycloalkyl, C₆-C₁₀-Aryl oder C₇-C₁₂-Aralkyl stehen, wobei alle diese Reste gegebenenfalls durch C₁-C₄-Alkoxy und/oder Halogen substituiert sein können. Die Reste R⁶ und R⁷, R⁷ und R⁸ oder R⁶, R⁷ und R⁸ können auch jeweils gemeinsam mit dem dazwischenliegenden C-Atom einen 5 bis 7 C-Atome enthaltenden Cyclus bilden.

Bevorzugte tertiäre Alkohole sind: tert.-Butanol, tert.-Amylalkohol, Methyldiethylcarbinol, Triethylcarbinol, 2-Methyl-pentanol-2, 2-Methylhexanol-2, 1-Methyl-cyclopentanol-1 und 1-Methylcyclohexanol-1. Besonders bevorzugt sind tert.-Butanol und tert.-Amylalkohol.

Die tertiären Alkohole können als solche oder im Gemisch mit inerten Lösungsmitteln eingesetzt werden. Beispiele für inerte Lösungsmittel sind: Aliphatische Kohlenwasserstoffe wie Cyclohexan, C₆-C₁₂-Alkane und Gemische davon; aromatische Kohlenwasserstoffe wie Benzol, Toluol, Xylole, Cumol, Diisopropylbenzole und Ethylbenzol; halogenierte Aromaten wie Chlorbenzol, Dichlorbenzole, Trichlorbenzole und Chlortoluole; Dialkyl-, Aralkyl- und Diarylether wie Diisopropylether, Methyl-tert.-butylether, Anisol, Phenetol und Diphenylether; cyclische Ether wie Tetrahydrofuran, Tetrahydropyran und 1,4-Dioxan und Sulfone wie Dimethylsulfoxid und Tetramethylensulfon.

Bezogen auf tertiären Alkohol kann man z.B. 0,01 bis 50 Gew.-% und vorzugsweise 0,1 bis 20 Gew.-% inertes Lösungsmittel zusetzen. Besonders bevorzugt werden tertiäre Alkohole ohne Zusätze von inertem Lösungsmittel eingesetzt.

Die tertiären Alkohole können z.B. in Mengen von 100 bis 5 000 g pro 100 g eingesetzter Verbindung der Formel (II) verwendet werden. Vorzugsweise wählt man die Menge an tertiärem Alkohol so, daß ein gut rührbares Reaktionsgemisch entsteht.

Das erfindungsgemäße Verfahren kann z.B. bei Temperaturen im Bereich -20 bis +200°C durchgeführt werden. Bevorzugt sind Temperaturen von 0 bis 100°C, insbesondere von 20 bis 50°C.

Das erfindungsgemäße Verfahren kann z.B. bei Drucken von 1 bis 100 bar durchgeführt werden. Wenn man das erfindungsgemäße Verfahren bei Temperaturen durchführen möchte, bei denen einzelne Bestandteile des Reaktionsgemisches bei Normaldruck gasförmig sind, so ist es erforderlich, in geschlossenen Gefäßen zumindest unter dem sich von selbst einstellenden Druck zu arbeiten. Häufig ist es vorteilhaft, inerte Gase wie Stickstoff, Helium oder Argon aufzudrücken und bei 5 bis 50 bar, insbesondere bei 10 bis 30 bar zu arbeiten.

Die Reaktionsdauer hängt im Einzelfall von der Zusammensetzung des Reaktionsgemisches und den Reaktionsbedingungen ab. Sie kann z.B. zwischen 1 und 48 Stunden betragen. Häufig liegt sie im Bereich 3 bis 30 Stunden und beim Arbeiten bei 10 bis 50°C häufig im Bereich 5 bis 24 Stunden.

Das nach Beendigung der erfindungsgemäßen Umsetzung vorliegende Reaktionsgemisch kann man beispielsweise so aufarbeiten, daß man zunächst, evtl. bei vermindertem Druck, tertiären Alkohol und gegebenenfalls vorhandenes inertes Lösungsmittel abtrennt, den Rückstand mit Wasser versetzt und das sich bildende kristalline Produkt absaugt, nachwäscht und trocknet. Wenn man es noch weiter reinigen will, kann man es umkristallisieren.

Eine bevorzugte Ausführungsform des erfindungsgemäßen Verfahrens wird im folgenden beispielhaft anhand der Umsetzung 2-Methyl-5-(4-chlorbenzyliden)cyclopentanon mit Methylchlorid/Kaliumiodid erläutert:

2-Methyl-5-(4-chlorbenzyliden)-cyclopentanon, tert.-Butanol und Kaliumiodid werden in einem Druckautoklaven vorgelegt, Kaliumhydroxid hinzugegeben und Methylchlorid unter Rühren aufgedrückt. Man rührt eine gewisse Zeit bei Raumtemperatur oder leicht erhöhter Temperatur nach. Dann destilliert man den größeren Teil des tert.-Butanols ab und versetzt die zurückbleibende Suspension mit Wasser. Das kristalline Produkt wird abgesaugt, nachgewaschen und getrocknet. Zur weiteren Reinigung wird es z.B. aus einem Alkohol umkristallisiert.

Es ist ausgesprochen überraschend, daß für die erfindungsgemäße Alkylierung von nicht besonders methylenaktiven Ketonen, Alkalihydroxide als Basen eingesetzt werden können, genügen doch sonst für diesen Zweck nicht einmal Alkalimetallalkoholate, die um ein Vielfaches basischer sind (siehe J. March, Advanced Organic Chemistry, 4th Edition, New York 1992 und die dort zitierte Literatur). Der häufiger zitierte Einsatz von Alkali-tert.-alkoxiden wie z.B. Kalium-tert.-butylat in tert.-Butanol kann nicht mit dem Einsatz von Kaliumhydroxid in tert.-Butanol verglichen werden, da das Gleichgewicht 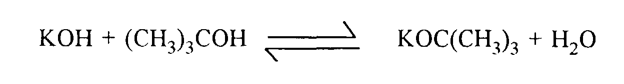 fast ganz auf der linken Seite liegt. Selbst im System Kalium-tert.-butylat/tert.-Butanol gelingt es nicht, das Keton vollständig ins Enolat überzuführen. Wenn das Enolation und unverändertes Keton nebeneinander vorliegen, sind in verstärktem Maße Nebenreaktionen zu erwarten (siehe Bull. Soc. Chim. Fr. 533, 537 (1950) und 1040 (1956)).

Der Einsatz der deutlich schwächeren Alkalimetallhydroxide sollte deshalb Nebenreaktionen stärker begünstigen. Umso überraschender ist es, daß die erfindungsgemäße Alkylierung mit Alkalimetallhydroxiden als Base trotzdem unter sehr milden Bedingungen und mit hohen Ausbeuten abläuft. Das erfindungsgemäße Verfahren ist eine äußerst einfach durchzuführende Methode zur Herstellung von 2,2-Dialkyl-aryliden-cycloalkanonen der Formel (I) in hohen Ausbeuten und unter milden Bedingungen.

Der Einsatz von schwierig handhabbaren Basen wie Metallhydriden oder Metallamiden, der nur mit erhöhtem Sicherheitsaufwand möglich ist, wird vermieden. Größere Mengen speziellere Lösungsmittel wie DMSO, DMF oder Ammoniak, die bei der Aufarbeitung des Reaktionsgemisches verwässert würden und anschließend aufwendig wiedergewonnen werden müßten, sind nicht erforderlich.

Die Möglichkeit des Einsatzes von Alkylchloriden in Gegenwart von Alkalimetalliodiden führt zu einer Kostenersparnis und vermeidet den Umgang mit Alkyliodiden, der wegen ihrer Cancerogenität besonderen Aufwand erfordert.

2,2-Dialkyl-aryliden-cycloalkanone der Formel (I) sind wertwolle Zwischenprodukte für die Herstellung von Pestiziden, insbesondere Fungiziden (siehe EP-A1 378 953, EP-A1 467 791 und EP-A1 467 792). Sie lassen sich auch durch Reduktion in 2,2-Dialkyl-benzyl-cycloalkanone überführen, die ihrerseits wieder als Ausgangsmaterialien für Fungizide dienen können (siehe EP-A1 267 778 und EP-A1 413 448).

### Beispiele

Prozentangaben sind Gewichtsprozente soweit nichts anderes vermerkt ist.

### Beispiel 1

### 2,2-Dimethyl-5-(4-chlorbenzyliden)-cyclopentanon

In einem 700 ml Edelstahl-Autoklaven wurden nacheinander 106 g 2-Methyl-5-(4-chlorbenzyliden)-cyclopentanon, 49,5 g 85 %ige wäßrige Kalilauge, 8,3 g Kaliumiodid und 400 g tert.-Butanol eingewogen und bei 20°C 50,5 g Methylchlorid aufgedrückt. Es wurde bei Raumtemperatur 23 Stunden gerührt und dann 300 ml tert.-Butanol bis zu einer Kopftemperatur von max. 50°C bei 20 mbar abdestilliert. Der Rückstand wurde mit 400 ml Wasser versetzt und gut verrührt. Die entstandene Suspension wurde über eine Nutsche abgesaugt, der Filterkuchen zweimal mit je 200 ml Wasser gewaschen und getrocknet. So erhielt man 112,8 g eines hellbeigefarbenen Feststoffes mit einem Gehalt von 89,5 %. Die Ausbeute des Rohproduktes betrug 90,2 % d. Th.

Zur Reinigung wurde das Rohprodukt in 250 ml Methanol aufgeschlämmt, für 30 min auf Rückflußtemperatur erhitzt und dann abgekühlt. Das abgeschiedene Produkt wurde filtriert, mit Methanol nachgewaschen und getrocknet. Man erhielt so 86 g reines Produkt mit einem Schmelzpunkt von 120 bis 122°C.

### Beispiel 2

### 2,2-Dimethyl-6-benzyliden-cyclohexanon

In einem 1 l 4-Halskolben wurden 66 g 85 %ige wäßrige Kalilauge und 750 ml tert.-Butanol vorgelegt und unter Rühren kurz zum Rückfluß erhitzt. Dann wurden 8,3 g Kaliumiodid zugegeben, auf Raumtemperatur abgekühlt und 200 g 2-Methyl-6-benzyliden-cyclohexanon zugegeben. Während 8 Stunden wurden 101 g Methylchlorid bei Raumtemperatur eingeleitet. Es wurde 8 Stunden bei Raumtemperatur nachgerührt und dann 450 ml tert.-Butanol abdestilliert. Der Rückstand wurde mit 500 ml Wasser versetzt, gut verrührt und das sich ausscheidende Produkt abgesaugt. Nach Umkristallisation aus Methanol erhielt man 161 g (75 % d.Th.) reines Produkt mit einem Schmelzpunkt von 79-81°C.

### Beispiele 3 bis 9

Es wurde analog Beispiel 1 verfahren und die folgenden Produkte hergestellt.

### Beispiel 3

2,2-Dimethyl-5-(4-fluorbenzyliden)-cyclopentanon, Schmelzpunkt 69°C.

### Beispiel 4

2,2-Diallyl-5-(4-chlorbenzyliden)-cyclopentanon, Öl.

### Beispiel 5

2,2-Dimethyl-5-(3,4-dichlorbenzyliden)-cyclopentanon, Öl.

### Beispiel 6

2-Ethyl-2-methyl-5-(4-chlorbenzyliden)-cyclopentanon, Schmelzpunkt 83-84°C.

### Beispiel 7

2,2-Dimethyl-5-(4-cyanobenzyliden)-cyclopentanon, Öl.

### Beispiel 8

2-Ethyl-2-methyl-6-(4-chlorbenzyliden)-cyclohexanon, Schmelzpunkt 71-72°C.

### Beispiel 9

2,2-Dimethyl-7-(4-chlorbenzyliden)-cycloheptanon, Schmelzpunkt 63°C.

## Patentansprüche

1. Verfahren zur Herstellung von 2,2-Dialkyl-aryliden-cycloalkanonen der Formel 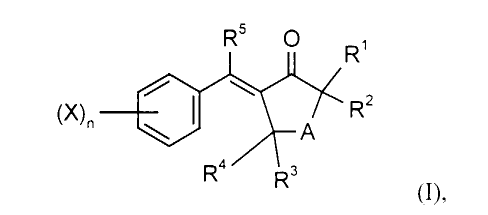 in der
A für gegebenenfalls substituiertes -(CH₂)-ₓ mit x = 1, 2 oder 3 steht,
R¹ und R² für gleiche oder verschiedene, gegebenenfalls substituierte C₁-C₄-Alkyl- oder C₃-C₇-Cycloalkylreste stehen,
R³, R⁴ und R⁵ gleich oder verschieden sind und für Wasserstoff oder gegebenenfalls substituiertes C₁-C₄-Alkyl oder C₃-C₇-Cycloalkyl stehen.
X für Halogen, Cyano, Nitro, C₁-C₄-Alkyl, C₁-C₄-Halogenalkyl, C₁-C₄-Alkoxy oder C₁-C₄-Halogenalkoxy stehen, wobei beim Vorhandensein mehrerer X-Reste diese gleich oder verschieden sein können und
n für null oder eine ganze Zahl von 1 bis 5 steht,
dadurch gekennzeichnet, daß man 2-Alkyl-aryliden-cycloalkanone der Formel 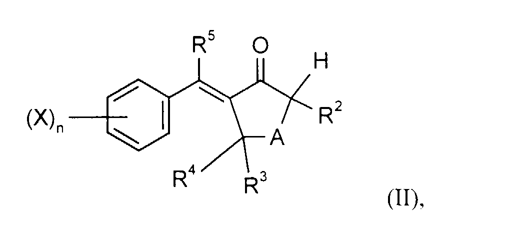 in der die verwendeten Symbole die bei Formel (I) angegebene Bedeutung haben, in Gegenwart von Metallhydroxiden und tertiären Alkoholen mit Alkylhalogeniden der Formel (III)
R1-Y (III)
in der
R¹ die oben angegebene Bedeutung hat und
Y für Chlor, Brom oder lod steht, umsetzt.

2. Verfahren nach Anspruch 1, dadurch gekennzeichnet, daß
A gegebenenfalls einen oder mehrere, gleiche oder verschiedene Substituenten aus der Gruppe C₁-C₄-Alkyl und C₃-C₇-Cycloalkyl enthält, wobei C₁-C₄-Alkyl seinerseits gegebenenfalls durch Halogen oder C₁-C₄-Alkoxy und C₃-C₇-Cycloalkyl seinerseits gegebenenfalls durch Halogen, C₁-C₄-Alkyl oder C₁-C₄-Alkoxy substituiert sein kann.
R¹ und R², soweit sie für C₁-C₄-Alkyl stehen, gegebenenfalls durch Halogen, C₁-C₄-Alkoxy, Halogen-C₁-C₄-alkoxy, C₂-C₄-Alkenyl, C₂-C₄-Alkenyl, Halogen-C₂-C₄-alkenyl oder Halogen-C₂-C₄-alkinyl substituiert sind und soweit R¹ und R² für C₃-C₇-Cycloalkyl stehen, dieses gegebenenfalls durch Halogen, C₁-C₄-Alkoxy oder C₁-C₄-Alkyl substituiert ist,
R³, R⁴ und R⁵, soweit diese für C₁-C₄-Alkyl stehen, gegebenenfalls durch Halogen oder C₁-C₄-Alkoxy und soweit sie für C₃-C₇-Cycloalkyl stehen, gegebenenfalls durch Halogen, C₁-C₄-Alkyl oder C₁-C₄-Alkoxy substituiert sind,
X für Fluor, Chlor, Cyano oder C₁-C₄-Alkyl und
n für null, 1 oder 2 stehen.

3. Verfahren nach Ansprüchen 1 und 2, dadurch gekennzeichnet, daß man als Verbindung der Formel (II) 2-Methyl- oder 2-Ethyl-5-(4-fluor-, 4-chlor-, 4-cyano- und 3,4-dichlor-benzyliden)-cyclopentanon, -cyclohexanon oder -cycloheptanon einsetzt.

4. Verfahren nach Anspruch 3, dadurch gekennzeichnet, daß man zusätzlich zu einem Alkylhalogenid der Formel (III) mit Y = Chlor 0,1 bis 50 Mol.-% eines Akaliiodids einsetzt (bezogen auf die Verbindung der Formel (II)).

5. Verfahren nach Ansprüchen 1 bis 4, dadurch geknnzeichnet, daß man Alkylhalogenide in Mengen von 1 bis 30 Molen bezogen auf l Mol eingesetzte Verbindung der Formel (II) einsetzt.

6. Verfahren nach Ansprüchen 1 bis 5, dadurch gekennzeichnet, daß es sich bei den Metallhydroxiden um Alkali- oder Erdalkalihydroxide handelt, die in mindestens äquimolaren Mengen eingesetzt werden.

7. Verfahren nach Ansprüchen 1 bis 6, dadurch gekennzeichnet, daß man als tertiäre Alkohole solche der Formel (IV) einsetzt 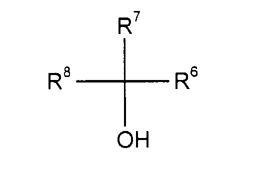 in der
R⁶, R⁷ und R⁸ gleich oder verschieden sind und jeweils für C₁-C₄-Alkyl, C₂-C₄-Alkenyl, C₂-C₄-Alkinyl, C₃-C₇-Cycloalkyl, C₆-C₁₀-Aryl oder C₇-C₁₂-Aralkyl stehen, wobei alle diese Reste gegebenenfalls durch C₁-C₄-Alkoxy und/oder Halogen substituiert sein können und die Reste R⁶ und R⁷oder R⁷ und R⁸ oder R⁶, R⁷ und R⁸ jeweils gemeinsam mit dem dazwischenliegenden C-Atom einen 5 bis 7 C-Atome enthaltenden Cyclus bilden können und man die tertiären Alkohole als solche oder im Gemisch mit inerten Lösungsmitteln und in Mengen von 100 bis 5 000 g pro 100 g Verbindung der Formel (II) einsetzt.

8. Verfahren nach Ansprüchen 1 bis 7, dadurch gekennzeichnet, daß man es bei Temperaturen im Bereich von -20 bis +200°C und bei Drucken im Bereich von 1 bis 100 bar durchführt.

9. Verfahren nach Ansprüchen 1 bis 8, dadurch gekennzeichnet, daß man das nach Beendigung der Umsetzung vorliegende Reaktionsgemisch so aufarbeitet, daß man zunächst tertiären Alkohol und gegebenenfalls vorhandenes inertes Lösungsmittel abtrennt, den Rückstand mit Wasser versetzt und das sich bildende kristalline Produkt absaugt, nachwäscht und trocknet.

## Claims

1. Process for the preparation of 2,2-dialkylarylidene-cycloalkanones of the formula 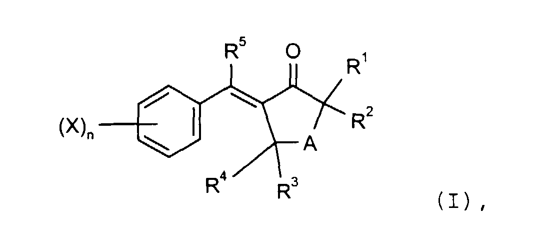 in which
A represents optionally substituted -(CH₂)-ₓ where x = 1, 2 or 3,
R¹ and R² represent identical or different, optionally substituted C₁-C₄-alkyl or C₃-C₇-cycloalkyl radicals,
R³, R⁴ and R⁵ are identical or different and represent hydrogen or optionally substituted C₁-C₄-alkyl or C₃-C₇-cycloalkyl,
X represents halogen, cyano, nitro, C₁-C₄-alkyl, C₁-C₄-halogenoalkyl, C₁-C₄-alkoxy or C₁-C₄-halogenoalkoxy, and, when two or more radicals X are present, they may be identical or different, and
n represents zero or an integer from 1 to 5,
characterized in that 2-alkyl-arylidene-cycloalkanones of the formula 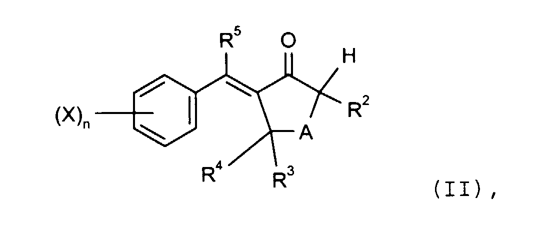 in which the symbols used have the meaning given for formula (I), are reacted in the presence of metal hydroxides and tertiary alcohols with alkyl halides of the formula (III)
R¹-Y (III)
in which
R¹ has the abovementioned meaning, and
Y represents chlorine, bromine or iodine.

2. Process according to Claim 1, characterized in that
A optionally contains one or more identical or different substituents from the group consisting of C₁-C₄-alkyl and C₃-C₇-cycloalkyl, it being possible for C₁-C₄-alkyl in turn to be optionally substituted by halogen or C₁-C₄-alkoxy and for C₃-C₇-cycloalkyl in turn to be optionally substituted by halogen, C₁-C₄-alkyl or C₁-C₄-alkoxy,
R¹ and R², where they represent C₁-C₄-alkyl, are optionally substituted by halogen, C₁-C₄-alkoxy, halogeno-C₁-C₄-alkoxy, C₂-C₄-alkenyl, C₂-C₄-alkinyl, halogeno-C₂-C₄-alkenyl or halogeno-C₂-C₄-alkinyl, and, where R¹ and R² represent C₃-C₇-cycloalkyl, the latter is optionally substituted by halogen, C₁-C₄-alkoxy or C₁-C₄-alkyl,
R³, R⁴ and R⁵, where they represent C₁-C₄-alkyl, are optionally substituted by halogen or C₁-C₄-alkoxy and, where they represent C₃-C₇-cycloalkyl, are optionally substituted by halogen, C₁-C₄-alkyl or C₁-C₄-alkoxy,
X represents fluorine, chlorine, cyano or C₁-C₄-alkyl and
n represents zero, 1 or 2.

3. Process according to Claims 1 and 2, characterized in that the compound of the formula (II) employed is 2-methyl- or 2-ethyl-5-(4-fluoro-, 4-chloro-, 4-cyano- and 3,4-dichloro-benzylidene)-cyclopentanone,-cyclohexanone or -cycloheptanone.

4. Process according to Claim 3, characterized in that, in addition to an alkyl halide of the formula (III) where Y = chlorine, from 0.1 to 50 mol-% of an alkali metal iodide is employed (based on the compound of the formula (II)).

5. Process according to Claims 1 to 4, characterized in that alkyl halides are employed in quantities of from 1 to 30 mol based on 1 mol of compound of the formula (II) employed.

6. Process according to Claims 1 to 5, characterized in that the metal hydroxides are alkali metal or alkaline earth metal hydroxides which are employed in at least equimolar quantities.

7. Process according to Claims 1 to 6, characterized in that the tertiary alcohols employed are those of the formula (IV) 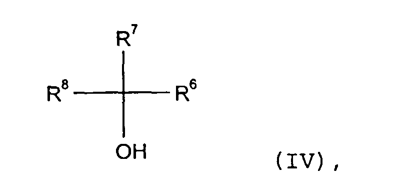 in which
R⁶, R⁷ and R⁸ are identical or different and each represent C₁-C₄-alkyl, C₂-C₄-alkenyl, C₂-C₄-alkinyl, C₃-C₇-cycloalkyl, C₆-C₁₀-aryl or C₇-C₁₂-aralkyl, all of which radicals can optionally be substituted by C₁-C₄-alkoxy and/or halogen, and the radicals R⁶ and R⁷ or R⁷ and R⁸ or R⁶, R⁷ and R⁸ can in each case form, together with the carbon atom lying between them, a ring containing 5 to 7 carbon atoms, and the tertiary alcohols are employed as such or as a mixture with inert solvents and in quantities of from 100 to 5000 g per 100 g of compound of the formula (II).

8. Process according to Claims 1 to 7, characterized in that it is carried out at temperatures in the range from -20 to +200°C and at pressures in the range from 1 to 100 bar.

9. Process according to Claims 1 to 8, characterized in that the reaction mixture which is present after the end of the reaction is worked up such that first of all tertiary alcohol and any inert solvent present are separated off, water is added to the residue, and the crystalline product which forms is filtered off with suction, washed and dried.

## Revendications

1. Procédé de préparation de 2,2-dialkyl-arylidène-cycloalcanones de formule 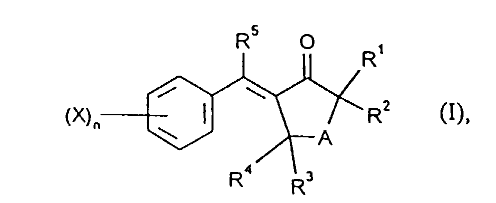 dans laquelle
A représente un reste -(CH₂)ₓ- éventuellement substitué, où x est égal à 1, 2 ou 3,
R¹ et R² représentent des restes alkyle en C₁-C₄ ou cycloalkyle en C₃-C₇ éventuellement substitués, identiques ou différents,
R³, R⁴ et R⁵ sont identiques ou différents et représentent l'hydrogène ou des restes alkyle en C₁-C₄ ou cycloalkyle en C₃-C₇ éventuellement substitués,
X représente un halogène ou un reste cyano, nitro, alkyle en C₁-C₄, halogénoalkyle en C₁-C₄, alcoxy en C₁-C₄ ou halogénoalcoxy en C₁-C₄, et, lorsque plusieurs restes X sont présents, ceux-ci peuvent être identiques ou différents, et
n représente 0 ou un nombre entier de 1 à 5,
caractérisé en ce que l'on fait réagir des 2-alkyl-arylidène-cycloalcanones de formule 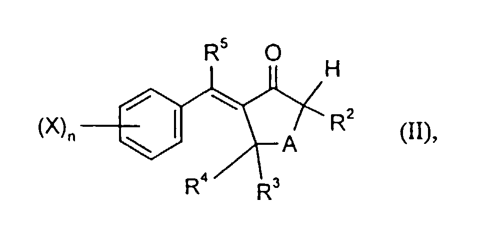 dans laquelle les symboles utilisés ont la signification indiquée pour la formule (I), en présence d'hydroxydes métalliques et d'alcools tertiaires, avec des halogénures d'alkyle de formule (III)
R¹-Y (III)
dans laquelle
R¹ a la signification indiquée ci-dessus, et
Y représente le chlore, le brome ou l'iode.

2. Procédé selon la revendication 1, caractérisé en ce que
A contient éventuellement un ou plusieurs substituants identiques ou différents du groupe constitué par les restes alkyle en C₁-C₄ et cycloalkyle en C₃-C₇, les restes alkyle en C₁-C₄ pouvant eux-mêmes être éventuellement substitués par halogène ou alcoxy en C₁-C₄ et les restes cycloalkyle en C₃-C₇ pouvant eux-mêmes être éventuellement substitués par halogène, alkyle en C₁-C₄ ou alcoxy en C₁-C₄,
R¹ et R², lorsqu'ils représentent des restes alkyle en C₁-C₄, sont éventuellement substitués par halogène, alcoxy en C₁-C₄, halogénoalcoxy en C₁-C₄, alcényle en C₂-C₄, alcynyle en C₂-C₄, halogénoalcényle en C₂-C₄ ou halogénoalcynyle en C₂-C₄, et, lorsque R¹ et R² représentent des restes cycloalkyle en C₃-C₇, ceux-ci sont éventuellement substitués par halogène, alcoxy en C₁-C₄ ou alkyle en C₁-C₄,
R³, R⁴ et R⁵, lorsqu'ils représentent des restes alkyle en C₁-C₄, sont éventuellement substitués par halogène ou alcoxy en C₁-C₄ et, lorsqu'ils représentent des restes cycloalkyle en C₃-C₇, ils sont éventuellement substitués par halogène, alkyle en C₁-C₄ ou alcoxy en C₁-C₄,
X représente le fluor, le chlore, ou un reste cyano ou alkyle en C₁-C₄, et
n est égal à 0, 1 ou 2.

3. Procédé selon les revendications 1 et 2, caractérisé en ce que l'on utilise comme composé de formule (II) une 2-méthyl- ou 2-éthyl-5-(4-fluoro-, 4-chloro-, 4-cyano- ou 3,4-dichloro-benzylidène)-cylopentanone, -cyclohexanone ou -cycloheptanone.

4. Procédé selon la revendication 3, caractérisé en ce que, en plus d'un halogénure d'alkyle de formule (III) dans laquelle Y est le chlore, on ajoute 0,1 à 50 % en mol d'un iodure de métal alcalin (par rapport au composé de formule (II)).

5. Procédé selon les revendications 1 à 4, caractérisé en ce que l'on utilise les halogénures d'alkyle en des quantités de 1 à 30 mol par mol de composé de formule (II) utilisé.

6. Procédé selon les revendications 1 à 5, caractérisé en ce que les hydroxydes métalliques sont des hydroxydes de métaux alcalins ou de métaux alcalino-terreux, que l'on utilise en des quantités au moins équimolaires.

7. Procédé selon les revendications 1 à 6, caractérisé en ce que l'on utilise comme alcools tertiaires des alcools de formule (IV) 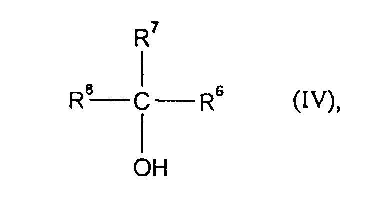 dans laquelle R⁶, R⁷ et R⁸ sont identiques ou différents et représentent chacun un reste alkyle en C₁-C₄, alcényle en C₂-C₄, alcynyle en C₂-C₄, cycloalkyle en C₃-C₇, aryle en C₆-C₁₀ ou aralkyle en C₇-C₁₂, tous ces restes pouvant être éventuellement substitués par alcoxy en C₁-C₄ et/ou halogène, et les restes R⁶ et R⁷ ou R⁷ et R⁸ ou R⁶, R⁷ et R⁸ peuvent à chaque fois former ensemble, avec l'atome de carbone auquel ils sont liés, un cycle contenant 5 à 7 atomes de carbone,
et en ce que l'on utilise les alcools tertiaires tels quels ou en mélange avec des solvants inertes et en des quantités de 100 à 5 000 g pour 100 g de composé de formule (II).

8. Procédé selon les revendications 1 à 7, caractérisé en ce qu'on l'effectue à des températures comprises entre -20 et + 200°C et sous des pressions comprises entre 1 et 100 bar.

9. Procédé selon les revendications 1 à 8, caractérisé en ce que l'on soumet le mélange réactionnel formé après la fin de la réaction à un traitement selon lequel on sépare d'abord l'alcool tertiaire et le solvant inerte éventuellement présent, on ajoute de l'eau au résidu et on filtre à la trompe le produit cristallin formé, on le lave et on le sèche.
